# EUROPEAN PATENT APPLICATION

(11) **EP 3 590 359 A1**
(43) Date of publication of application: **08.01.2020**
(21) Application number: 18305871.8
(22) Date of filing: 03.07.2018
(51) Int. Cl.: A23L 33/105, A23L 3/3499, A61K 36/53, A61K 36/537, A23K 20/111, A23K 20/10, A23L 13/40, A23L 15/00, A23L 17/00

(54) **TAXODIONE FOR ITS USE FOR PROTECTING MUSCLE AND MEAT FROM OXIDATION**

(71) Applicant: CENTRE NATIONAL DE LA RECHERCHE SCIENTIFIQUE, 75016 Paris (FR); UNIVERSITE DE MONTPELLIER, 34090 Montpellier (FR); INSTITUT NATIONAL DE LA SANTE ET DE LA RECHERCHE MEDICALE (INSERM), 75013 Paris (FR); Ecole Pratique des Hautes Etudes, 75014 Paris (FR); Universite Montpellier III Paul Valery, 34090 Montpellier (FR); Institut De Recherche Pour Le Développement (IRD), 13002 Marseille 2 (FR)
(72) Inventor: Saint, Nathalie, 34190 Brissac (FR); Rapior, Sylvie, 34090 Montpellier (FR); Vitou, Manon, 34470 Perols (FR); Bouguet, Guillaume, 34980 Saint Gely du Fesc (FR); Carnac, Gilles, 34000 Montpellier (FR); Morel, Sylvie, 34080 Montpellier (FR)
(74) Representative: Gevers & Orès

(57) **Abstract**

The present invention relates to the abietane diterpene taxodione and to rosemary stem extract containing taxodione for their use in treating a muscle wasting diseases and/or disorders; it also relates to the use of taxodione as natural meat preserver.

## Description

The present invention relates to the abietane diterpene taxodione for its use in treating a muscle wasting diseases and/or disorders; it also relates to the use of taxodione as natural meat preserver.

Indeed the Inventors have identified a compound in stems of *Rosmarinus officinalis* that can prevent the deleterious effects of oxidative stress in skeletal muscle cells. More specifically, they showed that taxodione protects human skeletal muscle cells from hydrogen peroxide-induced cytotoxic damage (by monitoring cell viability, ROS production, H2AX phosphorylation and *CHOP* gene expression, see experimental part) more efficiently than carnosic acid and carnosol, the two main reference antioxidant compounds of rosemary leaf extract. Moreover, they also showed that taxodione reduces lipid and protein oxidation in minced meat during refrigerated storage. Their study thus allowed to define taxodione as a cheap source of natural agent that can be used to limit oxidation in human skeletal muscle and processed meat.

Oxidative processes cause damages to biomolecules and are associated with muscle wasting diseases in humans, and undesirable changes in food systems (Canton, Menazza, & Di Lisa, 2014; Choi, Ow, Yang, & Taneja, 2016; Papuc, Goran, Predescu, & Nicorescu, 2017). In human and animal diseases, accumulation of pro-oxidant molecules derived from radical oxygen species (ROS) can affect the balance between protein synthesis and degradation, induces muscle fatigue, cell death and skeletal muscle repair dysfunction, resulting in extensive muscle loss over time (Canton, Menazza, & Di Lisa, 2014; Choi, Ow, Yang, & Taneja, 2016). During animal meat oxidation, changes in a large number of compounds, such as lipid peroxidation and discoloration (myoglobin oxidation), adversely affect aspect and quality of meat products and limit their shelf life (Papuc, Goran, Predescu, & Nicorescu, 2017). Antioxidant compounds can be used to prevent or delay these oxidative processes. Synthetic antioxidants have been added to meat and meat products with success, but their use has been discouraged because of their toxic effects and recent consumer interest in natural products.

Therefore, the meat industry is promoting research to identify new inexpensive and effective natural antioxidants (Shah, Bosco, & Mir, 2014). This research effort could be also useful for humans. Indeed, despite the clinical relevance of antioxidant treatments to improve skeletal muscle function and the great interest by the general population in antioxidant supplementation, evidences on their efficacy are very limited (Passerieux, Hayot, Jaussent, Carnac, Gouzi, Pillard, et al., 2015), and the antioxidant capacity to delay, prevent, or reverse loss of muscle mass is unclear (Steinhubl, 2008). Moreover, some antioxidants have deleterious effects on differentiation of skeletal muscle precursors (Ding, Choi, Kim, Han, Piao, Jeong, et al., 2008).

Therefore, there remains a need to identify effective and safe natural antioxidant molecules for food application and human health.

Plants are an important source of bioactive molecules (Newman & Cragg, 2012). Rosemary (*Rosmarinus officinalis* L., *Lamiaceae)* leaf extracts contain many different phenolic compounds, including flavonoids and phenolic diterpenes and triterpenes (Borras-Linares, Stojanovic, Quirantes-Pine, Arraez-Roman, Svarc-Gajic, Fernandez-Gutierrez, et al., 2014), with many major biological properties (antidiabetic, anti-inflammatory, antioxidant and anticancer) (Altinier, Sosa, Aquino, Mencherini, Della Loggia, & Tubaro, 2007; Bakirel, Bakirel, Keles, Ulgen, & Yardibi, 2008; Lo, Liang, Lin-Shiau, Ho, & Lin, 2002; Perez-Fons, Garzon, & Micol, 2010). The antioxidant activities of rosemary leaf extracts can mainly be attributed to two phenolic diterpenes carnosic acid (CA) and carnosol (CO), and to a lesser extent to other phenolic compounds, such as rosmarinic acid (Birtic, Dussort, Pierre, Bily, & Roller, 2015; Srancikova, Horvathova, & Kozics, 2014). Rosemary leaves extracts have been approved for use in the European Union as food additive E932 under the Regulation 1333/2008 of the European Parliament and Council. Rosemary-derived ingredients are also used in the formulation of many cosmetics. Rosemary-based diets and its active molecules, essentially carnosic acid, can enhance the antioxidant status of animal skeletal muscle (Ortuno, Serrano, Jordan, & Banon, 2016). *Rosmarinus officinalis* extracts have also been used as preservatives in processed meat to replace chemical antioxidants and protect from oxidation (Shah, Bosco, & Mir, 2014; Xiong, 2017). Moreover, rosemary essential oil was used to extend the shelf life of refrigerated meat (Sirocchi, Devlieghere, Peelman, Sagratini, Maggi, Vittori, et al., 2017).

The purpose of the studies conducted by the Inventors has been to identify new natural molecules that could be extracted from rosemary by-products, for instance the stems, and able to prevent the deleterious effects of oxidative stress in skeletal muscle cells for mammalian, in particular human health and in meat for food applications.

In this context, they identified taxodione as a highly efficient molecule able to prevent oxidation in biological complex media, in particular, in mammalian muscle.

According to a first embodiment, the present invention relates to taxodione or a rosemary stem extract for its use to prevent and/or decrease oxidation in muscle, more specifically oxidation occurring in skeletal muscle cells, in mammal, such as human, livestock and pets.

Taxodione (CAS 19026-31-4) is an abietane diterpene also named (4bS,8aS)-4b,5,6,7,8,8a-hexahydro-4-hydroxy-2-isopropyl-4b,8,8-trimethylphenanthrene-3,9-dione or 11-hydroxyabieta-7,9(11),13-triene-6,12-dione, having the following chemical structure:

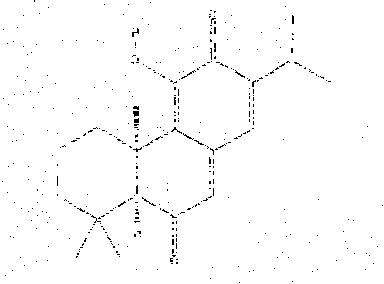

The taxodione (TX) was previously isolated from *Rosmarinus officinalis* roots with a purification yield of 0.14 mg/g of dry roots (Abou-Donia, Assaad, Ghazy, Tempesta, & Sanson, 1989), identified in stems (purification unspecified) (El-Lakany, 2004) and, isolated in mixture with [9]-shogaol in leaves (Borras-Linares, Perez-Sanchez, Lozano-Sanchez, Barrajon-Catalan, Arraez-Roman, Cifuentes, et al., 2015). TX was also described in different plants: *Taxodium distichum, Taxodium ascendens, Cupressus sempervirens, Volkameria eriophylla* (synonym: *Clerodendrum eriophyllum*)*, Plectranthus barbatus, Premna obtusifolia,* and several *Salvia* sp. Few studies have focused on obtaining large TX quantities: from *Taxodium distichum* seeds and cones (3-3.4 mg/g of dry matter) (Hirasawa, Izawa, Matsuno, Kawahara, Goda, & Morita, 2007; Kupchan, Karim, & Marcks, 1968), from *Salvia phlomoides* roots (3.72 mg/g of dry roots) (Hueso-Rodriguez, Jimeno, Rodriguez, Savona, & Bruno, 1983) and from transformed *Salvia austriaca* hairy roots (0.43 mg/g of dry roots and 1.15 mg/g by ultra-high-performance liquid chromatography-diode array detector-tandem mass spectrometry) (Kuzma, Wysokinska, Sikora, Olszewska, Mikiciuk-Olasik, & Szymanski, 2016) (Kuzma, Kaiser, & Wysokinska, 2017).

Taxodione has various bioactivities: antifungal, antimicrobiotic, antileishmanial, antiprotozoal, antifungal, human cholinesterase inhibitor, antioxidant and antiproliferative and pro-apoptotic in cancer cell lines (see table 1):

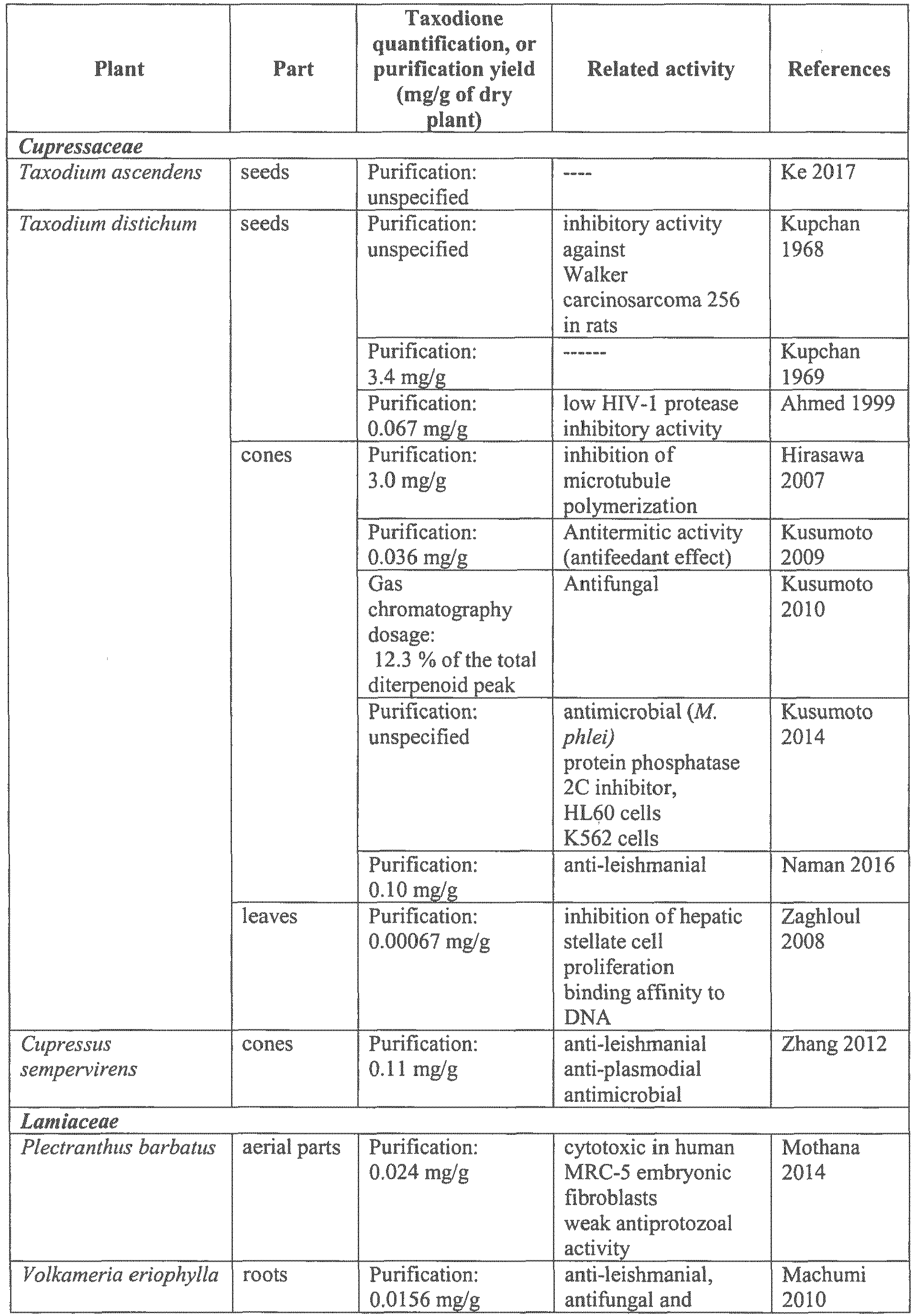

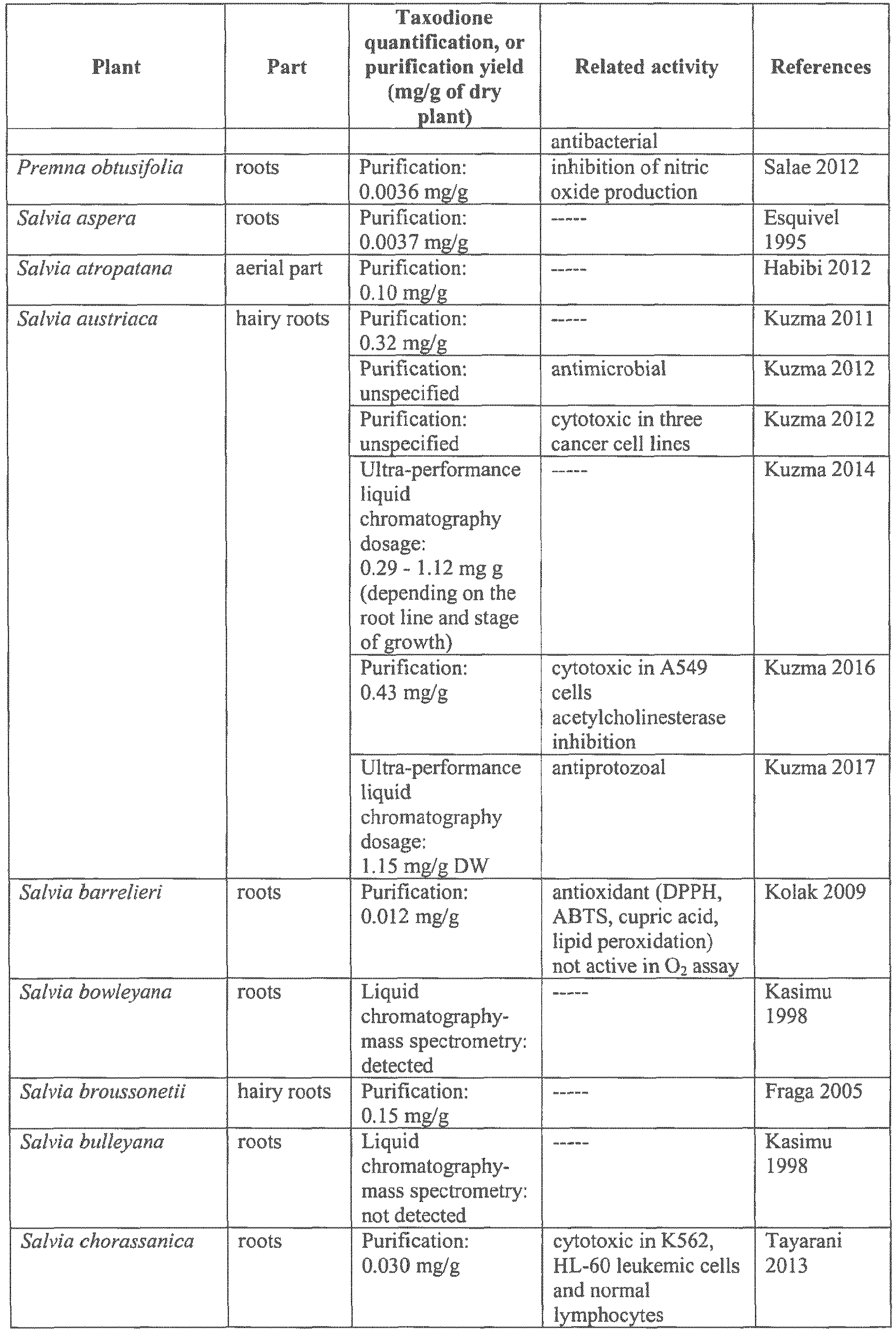

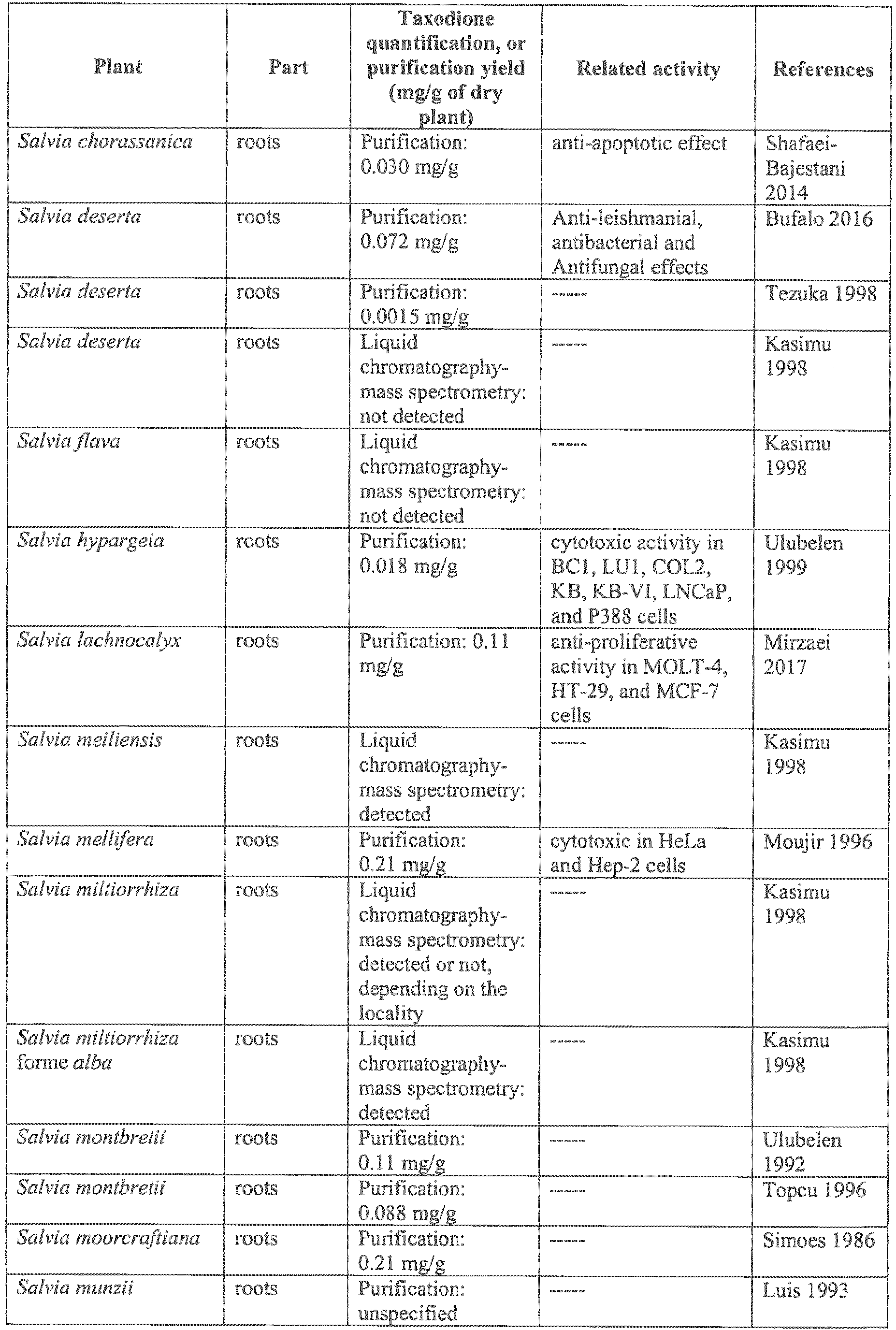

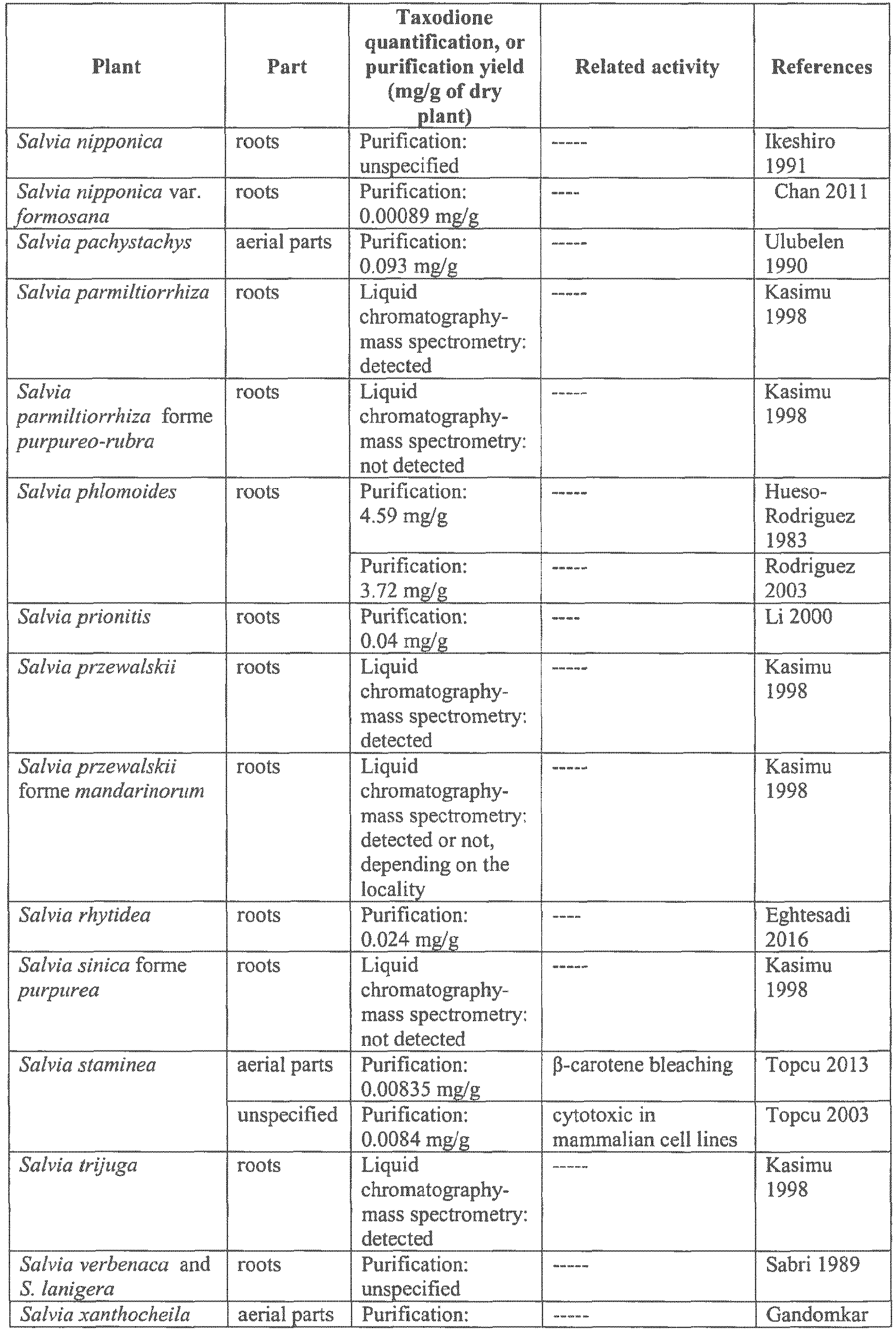

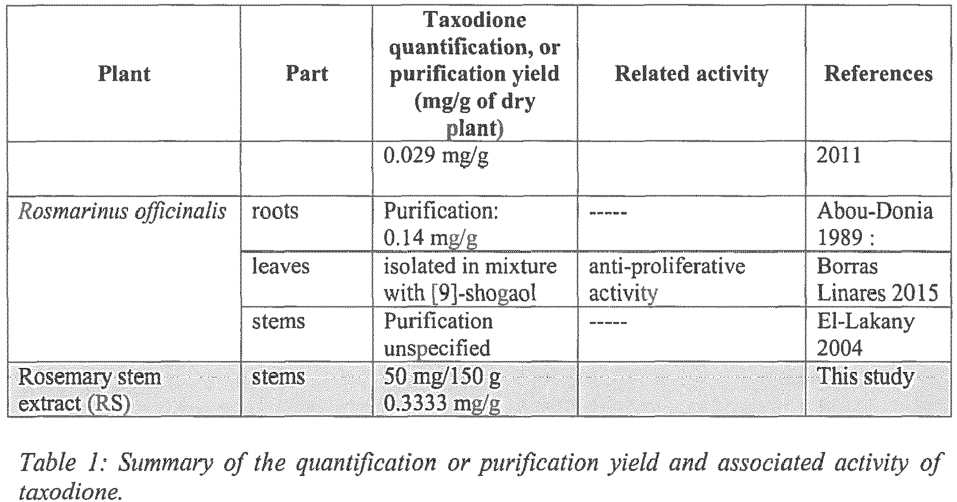

In the present invention, taxodione may be contained in a rosemary stem extract.

The present invention relates to taxodione or an extract of rosemary stem for its use to prevent and/or decrease proteins oxidative degradation and/or lipid oxidation in muscles and/or to prevent and/or decrease accumulation of pro-oxidant molecules in muscles.

Accordingly, the present invention relates to taxodione or an extract of rosemary stem for its use to prevent and/or treat loss of muscle mass and/or muscle fatigue and/or muscle wasting diseases wherein said loss of muscle mass, muscle fatigue and muscle wasting diseases are associated with oxidative stress.

Muscle wasting refers to the progressive loss of muscle mass and/or to the progressive weakening and degeneration of muscles, including the skeletal or voluntary muscles, which control movement, cardiac muscles, which control the heart (cardiomyopathies), and smooth muscles. Chronic muscle wasting is a chronic condition (i.e. persisting over a long period of time) characterized by progressive loss of muscle mass, and weakening and degeneration of muscle.

The loss of muscle mass that occurs during muscle wasting can be characterized by muscle protein degradation by catabolism. Protein catabolism occurs because of an unusually high rate of protein degradation, an unusually low rate of protein synthesis, or a combination of both. Muscle protein catabolism, whether caused by a high degree of protein degradation or a low degree of protein synthesis, leads to a decrease in muscle mass and to muscle wasting.

Muscle wasting is usually associated with ageing and chronic, neurological, genetic or infectious pathologies, diseases, illnesses or conditions. These include muscular dystrophies such as Duchenne muscular dystrophy, Becker muscular dystrophy, limb-girdle disease, and myotonic dystrophy, FacioScapuloHumeral dystrophy, laminopathies, dystrophies caused by mutations in the collagen VI and dysferlin genes; muscle atrophies such as post-polio muscle atrophy (PPMA); cachexias such as cardiac cachexia, AIDS cachexia and cancer cachexia; and malnutrition, leprosy, diabetes, renal disease, chronic obstructive pulmonary disease (COPD), cancer, end stage renal failure, sarcopenia, emphysema, osteomalacia, HIV infection, AIDS, and cardiomyopathy.

Muscle wasting, if left unabated, can have dire health consequences. For example, the changes that occur during muscle wasting can lead to a weakened physical state that is detrimental to an individual's health, resulting in increased susceptibility to bone fracture and poor physical performance status.

The present invention also relates to a method of treating, reducing the severity, reducing the incidence, delaying the onset, or reducing the pathogenesis of muscle wasting diseases associated with oxidative stress in a subject in need thereof, comprising the step of administering to said subject a therapeutically effective amount of taxodione or a rosemary stem extract.

In a particular embodiment of the invention, taxodione or the extract of rosemary stem is formulated in a pharmaceutical composition.

As used herein, a pharmaceutical composition comprises a therapeutically effective amount of the active ingredient, i.e. taxodione or extract of rosemary stem, together with a pharmaceutically acceptable carrier or diluent. A "therapeutically effective amount" as used herein refers to that amount which provides a therapeutic effect for a given condition and administration regimen.

As used herein, the term "administering" refers to bringing a subject in contact with a compound of the present invention.

The pharmaceutical compositions containing the compounds of this invention can be administered to a subject by any method known to a person skilled in the art, such as orally, parenterally, intravascularly, paracancerally, transmucosally, transdermally, intramuscularly, intranasally, intravenously, intradermally, subcutaneously, sublingually, intraperitoneally, intraventricularly, intracranially, intravaginally, by inhalation, rectally, intratumorally.

In one embodiment, the pharmaceutical compositions are administered orally, and are thus formulated in a form suitable for oral administration, i.e. as a solid or a liquid preparation. Suitable solid oral formulations include tablets, capsules, pills, granules, pellets, powders, and the like. Suitable liquid oral formulations include solutions, suspensions, dispersions, emulsions, oils and the like.

As used herein "pharmaceutically acceptable carriers or diluents" are well known to those skilled in the art. The carrier or diluent may be a solid carrier or diluent for solid formulations, a liquid carrier or diluent for liquid formulations, or mixtures thereof.

Because taxodione or extract of rosemary stem are able to prevent and/or decrease proteins oxidative degradation and/or lipid oxidation in muscles and/or to prevent and/or decrease accumulation of pro-oxidant molecules in muscles, they find valuable use as nutritional agent for livestock.

Consequently, in another embodiment, the present invention relates to the use of taxodione or of an extract of rosemary stem as nutritional agent for livestock for improving meat antioxidant status and thus meat and meat-derived food product quality.

Indeed, the supplementation of livestock animals feeding with taxodione or an extract of rosemary stem is effective in delaying lipid peroxidation and/or protein degradation of meat and in improving color, flavor and texture stabilities of the meat.

The present invention also relates to a method for improving livestock meat quality, including decreasing protein degradation and/or lipid peroxidation and/or improving color, flavor and/or texture stabilities of the meat or of the meat-derived food products, especially avoiding discoloration and rancidity of the meat or of the meat-derived food products, comprising the step of adding taxodione or an extract of rosemary stem to livestock feed.

In a particular embodiment, said taxodione is administered in a quantity comprised between 1 to 20 mg/kg feed.

Food preservatives can be divided into two categories, natural preservatives and synthetic preservatives, depending on the source. Because of the high cost of natural preservatives, they are not widely used in food processing for a long time. Artificial synthetic preservative such as sodium benzoate have been obtained in food processing because of their low price and good preservative effect. Widely used, however, studies have found that some synthetic preservatives have problems such as carcinogenicity, teratogenicity, and susceptibility to food poisoning. For example, benzoate may cause food poisoning, and nitrite and nitrate may be generated (carcinogenic nitrosamines).

It thus remains a need to find new natural preservative easy to produce at low cost.

In their studies, the Inventors have also shown that taxodione is very efficient for preventing and/or decreasing oxidation occurring in processed meat, in particular, oxidation of proteins and lipids contained in processed meat.

According to another embodiment, the present invention thus relates to the use of taxodione or a rosemary stem extract as a natural preservative agent for complex food product containing proteins and/or lipids, such as food product containing meat.

The natural preservative of the present invention may be sprayed, impregnated or coated with a food product containing meat to form a layer film, or incorporated into said food product, which can significantly extend the shelf life of said food product, the preservative can show anti-oxidation function.

According to the present invention, a "food product containing proteins" may be non-heat treated processed meat and heat-treated processed meat, processed fish and fishery products including mollusks and crustaceans, processed eggs and egg products, dehydrated milk...; more specifically, a "food product containing meat" encompasses fresh meat of any origin, beef, veal, lamb and sheep, pork, poultry, for example sliced, minced or ground meat, that may be intended to be packaged before selling, delicatessen such as pâté, ham and smocked ham, sausage,...; "food product containing lipids" encompasses seasoning, condiments, mustard, soups and broth sauces, fats and oils essentially free from water...

The present invention also relates to a process of preparation of a food product containing proteins and/or lipid, such as food product containing meat, having an improved shelf life, wherein said process comprises the step of adding taxodione or a rosemary stem extract to said food product containing proteins and/or lipids, such as food product containing meat.

The person skilled in the art may chose the quantity of taxodione or of rosemary stem extract to be added to the food product containing proteins and/or lipids; for example, said quantity may represent between 1 to 50 mg, preferably between 1 to 10 mg, of taxodione per kg of complex food product containing proteins.

In all the embodiments of the present invention, taxodione may be contained in a rosemary stem extract. Said rosemary stem extract comprises at least 1% w/w of taxodione; said extract preferably comprises at least 3% w/w, more preferably 5% w/w of taxodione.

Said extract is preferably obtained by preparing a dry powder of rosemary stem; macerating said dry powder in an organic, such as alcoholic, or hydroalcoholic, solvent for at least 5 days, preferably 7 days, said solvent being preferably an alcohol, a hydrocarbon, a halogenated hydrocarbon, acetone, ethyl acetate, water or a mixture of these solvents; recovering the liquid phase by evaporating the solvent. The solvent is preferably a C₁-C₄ alcohol such as ethanol or a mixture of water and C₁-C₄ alcohol, such as ethanol.

The concentration of taxodione in an extract can be determined as described in the experimental part below.

To further enhance advantageous properties of taxodione or rosemary stem extract, they may be associated with nutritional agents of interest, such as vitamins, minerals, antioxidants...

The invention can be further illustrated by the following examples.

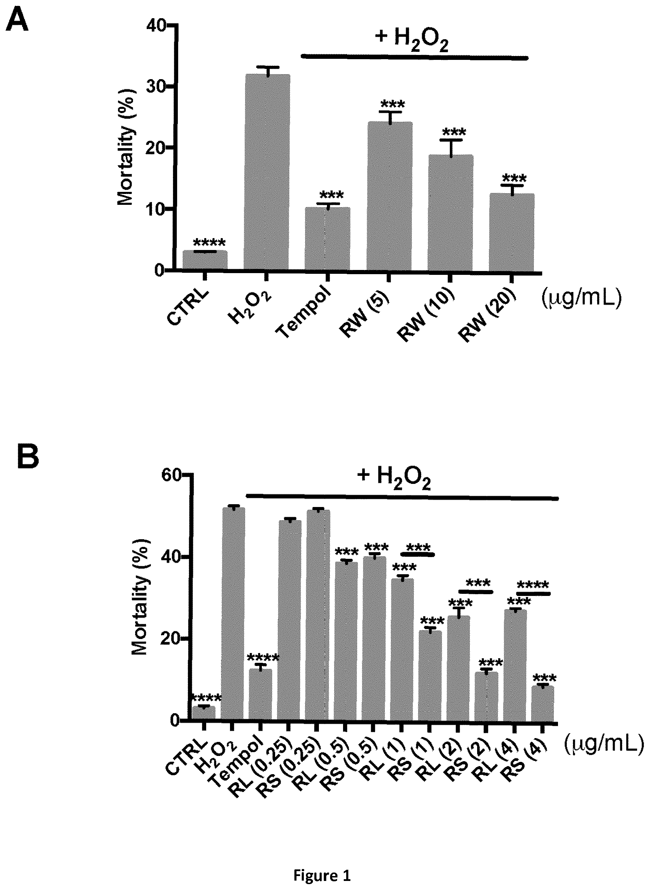

**Figure 1****: Rosemary stem extract protects human myoblasts from induced oxidative stress.**
   Cell death quantification (percentage of all cells) in human myoblasts that were incubated with (A) *Rosmarinus officinalis* whole extracts (RW) or tempol (synthetic antioxidant; 50 µM as control) or with (B) different concentrations of *Rosmarinus officinalis* leaf (RL) or stem (RS) extracts prior to incubation with (A,B) 120 µM H₂O₂ (lethal concentration). CTRL: cells not incubated with H₂O₂. Cell death was quantified using the Cell Count and Viability Kit and the Muse Cell Analyzer; p<0.001 (***) and p<0.0001 (****) compared with H₂O₂ (A, B) (one way ANOVA).
**Figure 2****: Different steps of taxodione purification from rosemary stem extract.**
**Figure 3****: Taxodione has a strong antioxidant activity on human muscle cells.**
   Cell death quantification (percentage of all cells) in human myoblasts upon incubation with the (A, B) indicated concentrations of taxodione (TX) or (B) of the main bioactive compounds of rosemary, carnosic acid (CA) and carnosol (CO), prior to exposure to (A, B) 120 µM H₂O₂ (lethal concentration). CTRL: cells not incubated with H₂O₂. Cell death was quantified using the Cell Count and Viability Kit and the Muse Cell Analyzer; p<0.05 (*), p<0.01 (**) and p<0.001 (***) compared with H₂O₂ (A, B) (one way ANOVA).

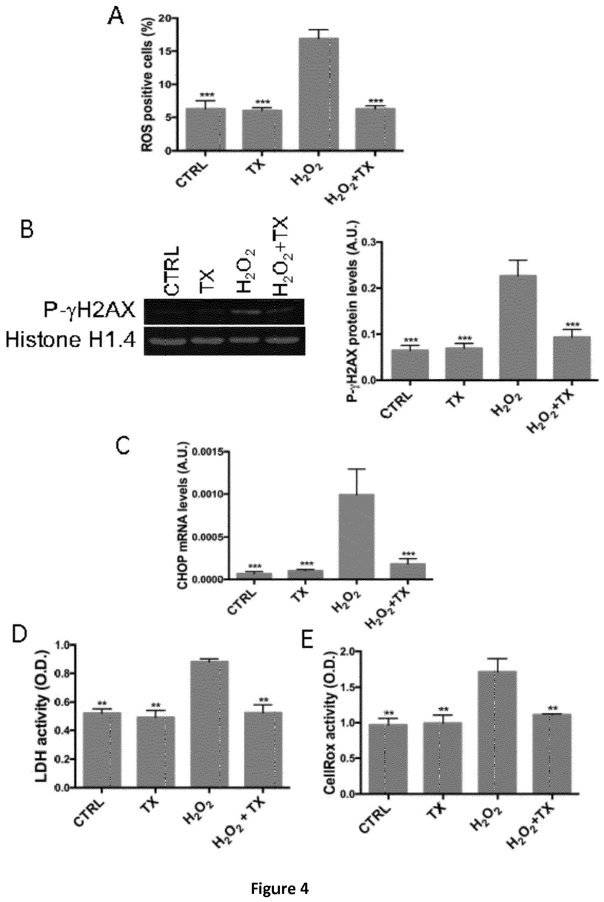

**Figure 4****: Taxodione decreases oxidative damage in human muscles cells.**
   Myoblasts were incubated with taxodione (TX) (0.5 µg/mL) for 24h prior to exposure to H₂O₂. (A) Reactive oxygen species (ROS) production was quantified with the "Muse oxidative stress Kit" and Fluorescence Activated Cell Sorting (FACS). (B) Western blot analysis of phosphorylated γH2AX protein level; histone H1.4 was used as loading control (right panel). Quantification of the Western blot data using the Odyssey software (left panel). (C) RT-qPCR analysis showing the relative expression levels (compared with untreated control) of the *CHOP* gene; *RPLPO* was used as reference gene. (D,E) Confluent human primary myoblasts were switched to differentiation medium for 4 days. At day 2, cells were incubated with TX (0.5 µg/mL) for 24h and then exposed to H₂O₂ for 24 h. (D) H₂O₂ toxicity was determined by quantifying lactate dehydrogenase (LDH) activity; (E) CellRox (ROS activity probe) was loaded in myotubes and fluorescence was quantified using a TECAN spectrophotometer; p<0.01 (**) and p<0.001 (***) compared with H₂O₂ (one way ANOVA).

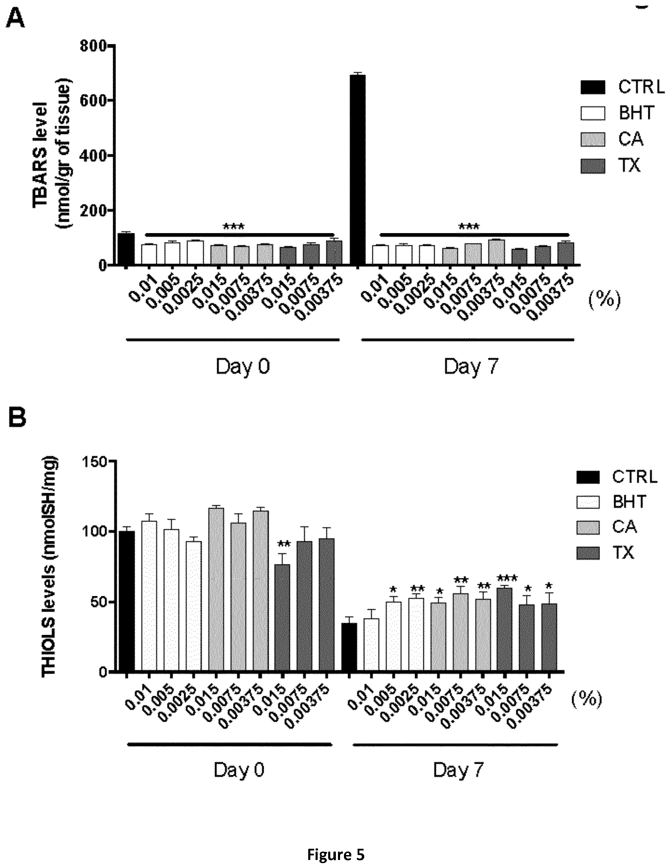

**Figure 5****: Taxodione protects minced meat from lipid and protein oxidation during refrigerated storage.**
   Minced gastrocnemius muscles from six-month-old C57BL/6 male mice were mixed with ethanol (CTRL) or BHT (0.010%, 0.005%, 0.0025% w/w minced muscle), carnosic acid (CA) (0.015%, 0.0075%, 0.00375% w/w minced muscle) or taxodione (TX) (0.015%, 0.0075%, 0.00375% w/w minced muscle) dissolved in ethanol. At day 0 and day 7 of refrigerated storage (+4°C), (A) lipid oxidation was evaluated by TBARS quantification, and (B) protein oxidation by total thiol quantification; p<0.05 (*), p<0.01 (**), p<0.001 (***) compared with CTRL (one way ANOVA).

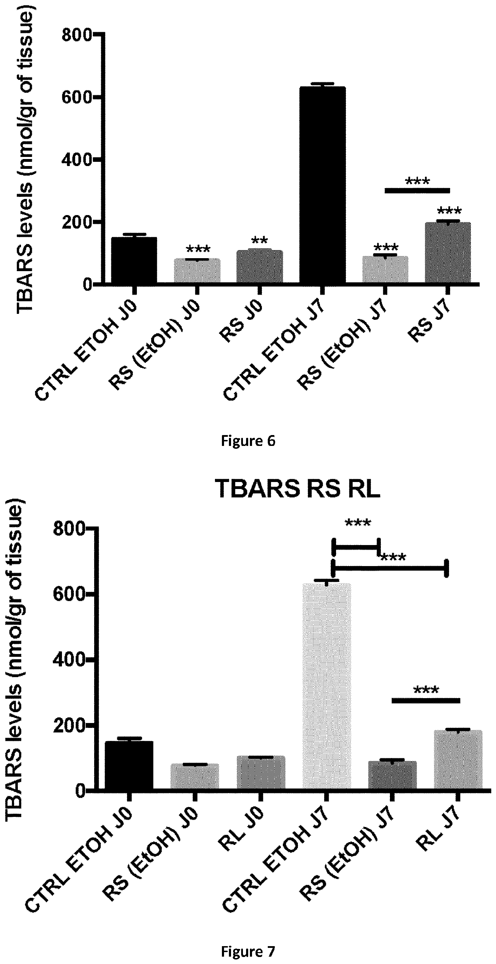

**Figure 6****: comparison of ethanolic rosemary stem extracts (RS (EtOH)) and hydroethanolic rosemary stem extracts (RS) on lipid oxidation of minced meat during refrigerated storage.**
**Figure 7****: comparison of rosemary stem extracts (RS) and rosemary leaf extracts (RL) on lipid oxidation of minced meat during refrigerated storage.**

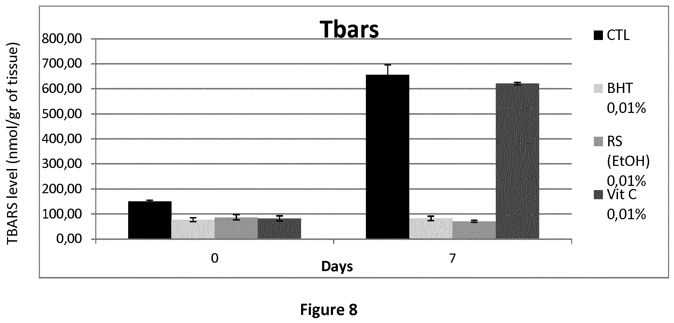

**Figure 8****: comparison of rosemary stem extracts (RS) and vitamin C on lipid oxidation of minced meat during refrigerated storage.**

### EXAMPLES

### Materials and Methods

### 1. General experimental procedure

Flash column chromatography was performed using a Spot Liquid Chromatography Flash instrument (Armen Instrument, Saint-Ave, France) equipped with an UV/visible spectrophotometer, a quaternary pump and a fraction collector. ¹H NMR, ¹³C NMR and 2D NMR spectra were recorded in the appropriate deuterated solvent on a BRUKER Avance III - 600 MHz NMR spectrometer.

### 2. Reagent and standards

DPPH radical (97%), cyclohexane (99.8%), chloroform (99%), dichloromethane (99.9%), deuterated chloroform (99.8%), DMSO (99.9%) and Tempol were purchased from Sigma-Aldrich (Steinheim, Germany). Acetonitrile (99.9%) was purchased from Chromasolv (Seelze, Germany). Formic acid (98%), ethyl acetate (99%) and acetone (99.5%) were from Panreac (Barcelona, Spain). Trolox (98%) was purchased from Fluka Chemicals (Steinheim, Switzerland), and ethanol (99.9%) from VWR BDH Prolabo (Pennsylvania, USA). L-ascorbic acid (Vitamin C) (Sigma-Aldrich, France)

### 3. Plant material

*Rosmarinus officinalis* was collected in the North of Montpellier (France) in February 2015. Dry stems and leaves were ground and directly extracted.

### 4. Extraction

150 g of ground rosemary stems were macerated in the dark at room temperature with 900 g of absolute ethanol and 450 g of distilled water, with agitation every 24 h. After 7 days, the stem extract was filtered. Evaporation under reduced pressure to dryness yielded 12.2 g of hydroethanolic extract, named RS (Rosemary Stems). The same procedure was used for 150 g of ground leaves and allowed obtaining 69 g of hydroethanolic extract, named RL (Rosemary Leaves). The same procedure was used for 150 g of ground leaves and stems, named RW (Rosemary Whole). A 100% ethanolic extract has also been prepared with the same procedure for 150g of ground stem; 5.3 g of extract, named RS (EtOH) has been obtained. The dry extracts were kept at -20 °C until analysis and purification.

### 5. Bioassay-guided isolation of taxodione from the rosemary stem extract

At each purification step, fractions were tested using the assays described below. The RS extract (12.2 g) was partitioned in CH₂Cl₂ soluble fraction and aqueous fraction. After evaporation under reduced pressure to dryness, these two fractions yielded 4.41 g of CH₂Cl₂ soluble extract and 7.79 g of aqueous soluble extract. The CH₂Cl₂ soluble extract was separated on normal-phase flash column chromatography (Merck Chimie SVF D26-SI60, 15-40 µm-30 g, flow rate 6.5 mL/min, 25 mL/fraction). Elution was completed with mixtures of cyclohexane:ethyl acetate (100:0 to 0:100), and then chloroform:methanol (100:0 to 80:20 in 1% then 5% stepwise). After thin-layer chromatography (TLC) analysis, the first fractions eluted with 100% cyclohexane (fractions 1-69) were combined and concentrated under reduced pressure, yielding fraction F1 (370 mg). F1 was purified on LH-20 Sephadex gel (2.4 × 38 cm, 40 g LH-20, elution: 100% dichloromethane to 100% methanol in 50% stepwise, then 100% acetone, 3 mL/fraction). Fractions 17 to 33 eluted with 100% CH₂Cl₂ were combined and concentrated under reduced pressure, yielding fraction F1-2 (160 mg). F1-2 was finally purified on reverse-phase flash column chromatography (Chromabond® Flash, RS4 C18, 4.3 g, flow rate: 5 mL/min, 25 mL/fraction). Elution was completed with a mixture of acetonitrile/water (50:50 to 100:0) and gave 111 fractions. Fractions 17 to 29 eluted with acetonitrile/water (60:40) were combined (F1-2-3) to give 50 mg of pure taxodione.

### 6. High-performance liquid chromatography (HPLC) analysis

Chromatographic separation and detection for quantitative analysis were performed on a SpectroSYSTEM® instrument that included a P4000 pump, a SCM1000 degasser, an AS3000 automatic sampler and an UV6000LP DAD detector (Thermo Fisher Scientific Inc., San Jose, USA). The system was operated using the ChromQuest software, version 5.0. Chromatographic separation was achieved on an ODS Hypersyl C18 column (250 mm × 4.6 mm, 5 µm, Thermo Fisher Scientific Inc., San Jose, USA), with a column temperature maintained at 30°C. Fractions were eluted at a flow rate of 1 mL/min (initial back pressure of approximately 105 bar), using solvent A (water/formic acid 99.9:0.1 v/v) and solvent B (acetonitrile). The gradient used for the analysis of standards and rosemary extracts was: 0-10 min, 85% A; 10-20 min, 85-65% A; 20-25 min, 65-30% A; 25-30 min, 30% A; 30-50 min, 30-20% A; 50-60 min, 20-10% A; 60-70 min, 10-85%; 70-80 min 85% A. The UV/vis spectra were recorded in the 200-400 nm range and chromatograms were acquired at 230, 280 and 330 nm. Identification of rosmarinic acid, carnosol, carnosic acid and rosmanol in the crude extracts and fractions was based on comparison with the retention times and UV spectra of commercial standards.

### 7. Quantification of taxodione by HPLC

***Linearity*/*work range:*** Standard curves were generated with increasing amounts of TX corresponding to a concentration range of 0.029 to 1 mg/mL (n = 3). Peak areas of taxodione were integrated and a calibration curve constructed. In regression analysis, curve fitting was deemed acceptable if the regression coefficient r was > 0.99.

***Limit of detectio**n*/*L****imit of quantification (LOD*/*LOQ):*** The LOD was defined as the sample concentration resulting in a response three times higher than the noise level. The LOQ was defined as the sample concentration resulting in a response ten times higher than the noise level.

***Taxodione recovery*** was assessed by sample analysis at three different concentrations (0.05, 0.4 and 0.8 mg/mL). Accuracy was expressed as percent error [(mean of measured)/mean of expected]x100, while precision was the determined coefficient of variation (CV, in %).

***Recovery in extract samples after addition of standard known amounts of taxodione:*** the RS extract was analysed by HPLC to quantify TX concentration and compared with the same extract spiked with known concentrations of pure TX. Recoveries were determined as [(mean value in the spiked extract - mean value in the not spiked extract)/(expected concentration) x 100].

### 8. Primary cultures of human myoblasts

The quadriceps muscle biopsy was from one healthy adult (AFM-BTR "Banque de tissus pour la recherche"). Myoblasts were purified from the muscle biopsy and were cultured on collagen-coated dishes in DMEM/F12 medium with 10% foetal bovine serum (FBS), 0.1% Ultroser G and 1 ng/ml of human basic fibroblast growth factor (proliferation medium), as previously described (Kitzmann, Bonnieu, Duret, Vemus, Barro, Laoudj-Chenivesse, et al., 2006). For cell differentiation, confluent cells were cultured in DMEM with 4% FBS for 3-5 days (differentiation medium).

### 9. Cell death and ROS quantification

Myoblasts: Myoblasts were seeded in 35 mm collagen-coated dishes, cultured in proliferation medium, pre-incubated or not with the tested compounds for 24 h and then incubated or not with a lethal concentration of hydrogen peroxide (H₂O₂), a strong pro-oxidant/pro-apoptotic compound, for 24 h. The optimal H₂O₂ concentration was the concentration required to kill between 30% and 50% of all cells and was established before each experiment. In general, myoblasts were incubated with 120 µM H₂O₂. Dead myoblasts were identified by staining with the Muse® Count and Viability Kit, and ROS was quantified with the Muse® Oxidative Stress Kit, followed by analysis with a Fluorescence Activated Cell Sorting (FACS) Muse apparatus (Millipore, France). Myotubes: Myoblasts were seeded in 35 mm collagen-coated dishes, cultured in proliferation medium until confluence, and then switched to differentiation medium for 4 days. At day 2, cells were incubated with TX for 24 h prior to incubation with H₂O₂ for 24 h. The H₂O₂ concentration used in myotube cultures (550 µM) was higher than that used for myoblasts, suggesting that myotubes are resistant to apoptosis inducers (unpublished results; (Salucci, Burattini, Baldassarri, Battistelli, Canonico, Valmori, *et al.,* 2013)). As myotubes are too big for FACS analysis, H₂O₂ effect was determined by quantifying lactate dehydrogenase (LDH) activity, which is increased in the culture medium during tissue damage, using the LDH Cytotoxic Kit (ThermoFisher, France). In parallel, myotube cultures were loaded with a ROS-fluorescent probe (CellRox) followed by fluorescence quantification using a TECAN spectrophotometer.

### 10. RT-qPCR assays

Myoblasts were seeded in 35 mm collagen-coated dishes, cultured in proliferation medium, pre-incubated or not with TX for 24 h, and then incubated or not with a sub-lethal concentration of H₂O₂ (80 µM; to avoid interference with dead cells) for 24 h. Then, total RNA was isolated from muscle cells using the NucleoSpin RNA II Kit (Macherey-Nagel, Hoerdt, France). The RNA concentration of each sample was measured with an Eppendorf BioPhotometer. cDNA was prepared using the Verso cDNA Synthesis Kit (Thermo Scientific, Ilkirch, France).

The expression of the *CHOP* (target) and *RPLPO* (control) genes was analysed by quantitative polymerase chain reaction (qPCR) on a LightCycler apparatus (Roche Diagnostics, Meylan, France), as previously described (El Haddad, Notarnicola, Evano, El Khatib, Blaquiere, Bonnieu, et al., 2017), using the following primers:
RPLPO:
   SEQ. ID. N°1: TCATCCAGCAGGTGTTCG
   SEQ. ID. N°2: AGCAAGTGGGAAGGTGTAA
CHOP:
   SEQ. ID. N°3: AAGGAAAGTGGCACAGC
   SEQ. ID. N°4: ATTCACCATTCGGTCAATCAGA.

### 11. Western blotting

Myoblasts were seeded in 35mm collagen-coated dishes, cultured in proliferation medium, pre-incubated or not with TX for 24 h and then incubated or not with 80 µM H₂O₂ for 24 h. Protein extracts were separated by SDS-PAGE gel electrophoresis and transferred to nitrocellulose membranes, blocked at room temperature with Odyssey blocking buffer (Eurobio, France) and probed with the rabbit polyclonal anti-Histone HI.4 (Sigma-Aldrich; 1/5000) and rabbit polyclonal anti-gamma H2AX (Cell signalling; 1/3000) antibodies followed by IRDye® 680RD and IRDye® 800RD secondary antibodies (Eurobio, France). Fluorescence was quantified with the Odyssey software. Data were normalized to α-tubulin expression.

### 12. Muscle sampling and preparation

The experimental protocol of this study was in strict accordance with the European directives (86/609/CEE) and was approved by the Ethical Committee of the Languedoc Roussillon Region. Gastrocnemius muscles from six-month-old C57BL/6 male mice were removed and immediately placed on ice. Muscles were then minced with sterile scissors for 5 min and divided in 600 mg batches. Each batch of minced muscle was mixed with different amounts of butylated hydroxytoluene (BHT) (0.010%, 0.005%, 0.0025% w/w minced muscle), CA (0.015%, 0.0075%, 0.00375% w/w minced muscle) or TX (0.015%, 0.0075%, 0.00375% w/w minced muscle) dissolved in ethanol (50 µL/600 mg). A control batch was mixed only with ethanol (50 µL/600 mg). Different percentages of the three antioxidants were used to correct for the molecular weight differences. Each batch of minced muscle was divided in four portions (150 mg) using a weighing cup, and individually packaged in polypropylene film bags. Three portions were stored at 4 ± 1°C in the dark for 7 days. The last one (0 day) was immediately homogenized in 50mM phosphate buffer (pH 7.0) (1:9) with an Ultra-Turrax homogenizer. The fraction of homogenate needed for thiobarbituric acid reactive substances (TBARS) measurement was quickly frozen, and the rest was centrifuged at 1000 g at 4 °C for 15 min before storage at - 20°C for total thiols measurements.

### 13. α, α-diphenyt-β-picrythydrazyl (DPPH) free radical scavenging assay

Radical scavenging activity was evaluated using DPPH according to the method described by Morel et al. with some modifications (Morel, Landreau, Nguyen, Derbre, Grellier, Pape, et al., 2012). Tested extracts and standards were diluted in absolute ethanol at different concentrations. Ethanol was used as blank, and 10, 25, 50 and 75 µM Trolox were used as calibration solutions. Tested compounds or standard solutions (100 µL) were placed in 96-well plates in triplicate for each tested concentration. Absolute ethanol was added (75 µL). The reaction was initiated by adding 25 µL of freshly prepared DPPH solution (1 mM) to obtain a final volume of 200 µL/well. After 30min in the dark at room temperature, absorbance was determined at 550nm with a UVMAX Molecular Devices microtiter plate reader (MDS Inc., Toronto, Canada). Results were expressed as the effective concentration at which 50% of DPPH radicals were scavenged (EC₅₀ in µg/mL). The results are the mean ± standard deviation (SD) of three independent experiments (three wells per concentration for each experiment).

### 14. TBARS measurement

The lipid peroxidation index was determined in muscle homogenates by measuring TBARS (Sunderman, Marzouk, Hopfer, Zaharia, & Reid, 1985). Briefly, muscle homogenates were mixed with 154 mM KCl, phosphoric acid (1% v/v) and 30 mM thiobarbituric acid (TBA). The mixture was boiled at 100°C for 1h. After cooling, it was extracted with n-butanol and centrifuged at 1000 g at room temperature for 15 min. The fluorescence intensity of the organic phase was measured with a spectrofluorometer (Ex: 515 nm; Em: 553 nm). A standard was prepared from 1,1,3,3-tetraethoxypropane (TEP), and results were expressed as nanomoles of TBARS per gram of tissue and were the mean ± SD of three experiments.

### 15. Protein oxidation assay or sulfhydryl group measurement

Total thiol quantification (Faure & Lafond, 1995) was based on the reaction of 5,5'-dithiobis (2-nitrobenzoic) (DTNB) with the samples that produces thionitrobenzoic acid (TNB), a yellow product that can be quantified spectrophotometrically at 412 nm. Results were expressed as nanomoles of total thiols per milligram of protein and were the mean ± SD of three experiments. Protein concentrations were determined using the BioRad Protein Assay (BioRad, Hercules, CA, USA) and bovine serum albumin as standard.

### 16. Statistical analysis

Statistical analysis was done with the GraphPad Prism 6.0 software (GraphPad Software Inc., San Diego, CA, USA). All experiments were performed in triplicate. Error bars represent the SD of the mean. Statistical significance was determined using one way ANOVA; p<0.05 (*), p<0.01 (**), p<0.001 (***) and p<0.0001 (****) were considered significant.

### Results and discussion

### 1. Rosemary stem extract has a strong antioxidant activity in complex biological system

H₂O₂, a strong pro-oxidant molecule, has previously been demonstrated to increase the percentage of apoptotic cells in adherent cultures of human myoblasts (skeletal muscle precursors) (Jean, Laoudj-Chenivesse, Notarnicola, Rouger, Serratrice, Bonnieu, et al., 2011).

The effect of pre-incubating human myoblasts with increasing concentrations of *Rosmarinus officinalis* extract from a mixture of leaves and stems (whole rosemary extract, RW) or Tempol, a powerful synthetic antioxidant, has been tested for 24h prior to incubation with a lethal concentration of H₂O₂. As expected, Tempol protected human myoblasts efficiently against H₂O₂-induced cell death (Fig. 1A). RW also efficiently reduced cell death at all tested concentrations.

Then, *Rosmarinus officinalis* leaf (RL) or stem (RS) extracts have been prepared and myoblasts have been incubated with increasing concentrations of RL or RS extracts below 10 µg/mL for 24h before addition of H₂O₂ and cell death quantification. RS was the most efficient in protecting myoblasts against H₂O₂-induced cell death at 1, 2 and 4 µg/mL (Fig. 1B). This result was quite surprising because the two main known rosemary antioxidants carnosic acid (CA) and carnosol (CO) are mainly extracted from leaves and are present at very low levels in the woody parts of the plant, such as stems (del Baño, Lorente, Castillo, Benavente-García, del Río, Ortuño, et al., 2003). This suggested that other molecule(s) might contribute to RS antioxidant activity.

### 2. Bioassay-guided isolation of the antioxidant compound from the RS extract

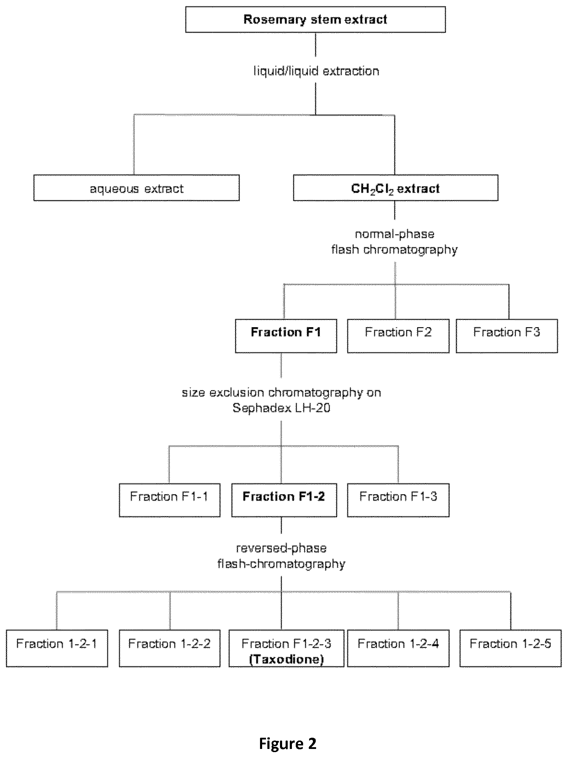

To isolate the compound(s) responsible for the antioxidant activity of the RS extract, a bioassay-guided fractionation approach has been used. Specifically, the RS extract has been separated in CH₂Cl₂ and water fractions (Fig. 2) and evaluated their ability to protect myoblasts against H₂O₂-induced cell death. This approach demonstrated that the CH₂Cl₂ soluble fraction was responsible for RS antioxidant activity (data not shown). Therefore, this fraction has been further fractionated (see Fig. 2 and Methods) to obtain 50 mg of pure compound. NMR and mass spectrometry analysis identified this compound as taxodione (TX) (Rodriguez, 2003), with a purification yield of 0.33 mg of taxodione (TX)/g of dry stems or 4.1 mg/ g dry extract.

To quantify TX in RS and RL extracts, a method has been developed and then validated by HPLC; this method indicated that in the RS extract, TX concentration was 11.7 mg/g dry extract, whereas it was undetectable in the RL extract (<LOD). In RS (EtOH), TX concentration was 38 mg/g dry extract. Quantification by HPLC suggested that TX concentration in the RS extract was higher than what suggested by the purification yield, implying that the conditions of extraction and purification can be improved.

### 3. Taxodione protects human myoblasts and myotubes against H₂O₂ induced stress

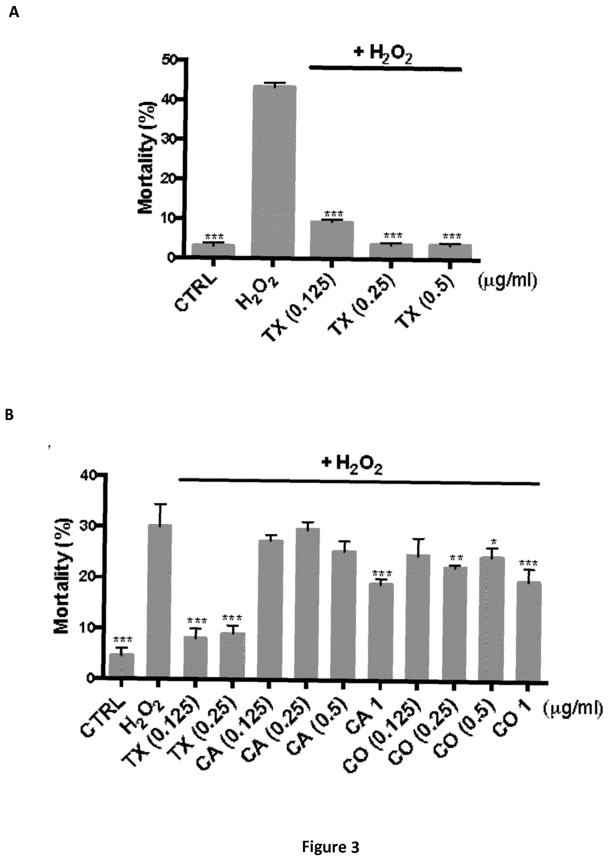

Myoblasts were incubated with 0.125 µg/mL, 0.250 µg/mL and 0.5 µg/mL of TX for 24 h before H₂O₂ addition. All three concentrations had similar and strong protective effect against H₂O₂-induced cell death (Fig. 3A).

TX antioxidant activity has then been compared with that of the main bioactive compounds of rosemary: CA and CO (Fig. 3B). TX was significantly more efficient at all tested concentrations -whereas Inventors had found that TX displayed low DPPH free radical scavenging activity-, compared with CA, CO and rosmarinic acid that showed strong antioxidant capacities like Trolox, as previously reported (Erkan, Ayranci, & Ayranci, 2008; Luis & Johnson, 2005).

Pro-oxidant molecules, such as H₂O₂, promote ROS production, DNA damage, reticulum endoplasmic stress, and cell differentiation alterations. Therefore, TX capacity to efficiently protect myoblasts against H₂O₂ damage has been assessed. After pre-incubation with TX for 24 h and exposure to H₂O₂ for 24 h, the level of ROS has been quantified (Fig. 4A), of γH2AX, a protein phosphorylated upon DNA double-strand break formation (Fig. 4B), and of the *CHOP* gene, a marker of endoplasmic reticulum stress (Fig. 4C).

As expected, H₂O₂ treatment increased the levels of ROS, γH2AX proteins and *CHOP* mRNA. Pre-treatment with TX reduced H₂O₂ effects, whereas TX alone did not have any effect. During muscle cell differentiation, myoblasts, the progeny of satellite stem cells, exit the cell cycle and spontaneously differentiate into myotubes that are quiescent multinucleated cells expressing muscle-specific structural proteins. To determine whether TX displayed antioxidant activity also in more mature skeletal muscle cells, we switched confluent human primary myoblasts to differentiation medium for 4 days. At day 2, we incubated cells with TX for 24h, followed by H₂O₂ for another 24h. LDH activity and ROS level were increased in myotubes incubated only with H₂O₂ (Fig. 4D, E). Conversely, pre-incubation with TX significantly reduced H₂O₂ effects.

It has thus been demonstrated that TX protects efficiently human skeletal muscle cells against oxidative stress. This suggests that TX could be useful in human pathologies associated with oxidative stress and skeletal muscle wasting diseases. It could also improve the efficacy of therapeutic approaches in skeletal muscle diseases by reducing the strong oxidative stress associated with these conditions.

### 4. Taxodione limits lipid and protein oxidation in minced meat.

In processed meat, lipids and proteins undergo oxidation over time, but this process can be delayed by addition of antioxidants (Shah, Bosco, & Mir, 2014).

Experiments on post-mortem meat from mice to characterize the antioxidant potential of TX have been developed.

As shown in meat for food, the lipid oxidation quantified by TBARS gradually increases in mouse muscles from the second day of storage at 4°C while the thiol levels decrease sharply indicating a high level of protein oxidation (data not shown). To determine TX antioxidant potential, the efficacy in decreasing lipid and protein oxidation of TX, CA and of the synthetic phenolic antioxidant BHT; of RS and RL and of RS, BHT, and vitamin C has been compared (Fig. 5, Fig. 7 and Fig. 8, respectively).

In minced mouse meat (CTRL), lipid oxidation, quantified by TBARS analysis, strongly increased after 7 days of storage at 4°C. Conversely, thiol levels dropped markedly, indicating a high level of protein oxidation (Fig. 5A and B). In meat samples containing BHT, CA or TX, TBARS values were already significantly lower at day 0 (Fig. 5A) and remained lower than in control (CTRL; non-treated samples) even at day 7 (Fig. 5A). At day 0, thiol levels were comparable in control and samples with BHT, CA or TX, but not for the sample with the highest TX concentration (0.015%) where total thiol level was significantly lower (Fig. 5B). After 7 days of storage, thiol level in meat was significantly lower in control than in the samples with antioxidants, but not for 0.01% BHT (Fig. 5B). The antioxidant capacity of extract of ground rosemary stems extracted in hydro-ethanolic (RS) or ethanolic (RS (EtOH)) buffer has been compared. Ethanolic extract contains more taxodione (2.86% of taxodione; quantification by HPLC-UV at 330nm) than the hydro-ethanolic extract (1.17% of taxodione). At day 0, the meat samples are characterized by TBARS values significantly decreased by the addition of RS or RS (EtOH) (Figure 6). At 7j, RS or RS (EtOH) treated samples maintained TBARS values at very low levels compared to the control group at the same day (Figure 6). However, the "ethanolic" extract of rosemary stems is significantly more effective than the "hydro-ethanolic" extract.

In addition, these assays also show an inhibition of lipid oxidation in meat of RS (EtOH) significantly improved compared to the inhibition of lipid oxidation in meat of RL (Fig. 7) and of vitamin C (Fig. 8).

These results show a protective effect of TX on lipid and protein oxidation during meat storage comparable to that of BHT and CA and RS (EtOH) better than RL and vitamin C.

### Bibliography

Abou-Donia, A., Assaad, A. M., Ghazy, N. M., Tempesta, M. S., & Sanson, D. R. (1989). Diterpene quinones from the roots of Rosmarinus officinalis L. Alexandria Journal of Pharmaceutical Sciences, 3(1), 54-55.
Altinier, G., Sosa, S., Aquino, R. P., Mencherini, T., Della Loggia, R., & Tubaro, A. (2007). Characterization of topical antiinflammatory compounds in Rosmarinus officinalis L. J Agric Food Chem, 55(5), 1718-1723.
Bakirel, T., Bakirel, U., Keles, O. U., Ulgen, S. G., & Yardibi, H. (2008). In vivo assessment of antidiabetic and antioxidant activities of rosemary (Rosmarinus officinalis) in alloxan-diabetic rabbits. J Ethnopharmacol, 116(1), 64-73.
Birtic, S., Dussort, P., Pierre, F. X., Bily, A. C., & Roller, M. (2015). Carnosic acid. Phytochemistry, 115, 9-19*.*
Borras-Linares, I., Perez-Sanchez, A., Lozano-Sanchez, J., Barrajon-Catalan, E., Arraez-Roman, D., Cifuentes, A., Micol, V., & Carretero, A. S. (2015). A bioguided identification of the active compounds that contribute to the antiproliferative/cytotoxic effects of rosemary extract on colon cancer cells. Food Chem Toxicol, 80, 215-222.
Borras-Linares, I., Stojanovic, Z., Quirantes-Pine, R., Arraez-Roman, D., Svarc-Gajic, J., Fernandez-Gutierrez, A., & Segura-Carretero, A. (2014). Rosmarinus officinalis leaves as a natural source of bioactive compounds. Int J Mol Sci, 15(11), 20585-20606.
Canton, M., Menazza, S., & Di Lisa, F. (2014). Oxidative stress in muscular dystrophy: from generic evidence to specific sources and targets. J Muscle Res Cell Motil, 35(1), 23-36.
Choi, M. H., Ow, J. R., Yang, N. D., & Taneja, R. (2016). Oxidative Stress-Mediated Skeletal Muscle Degeneration: Molecules, Mechanisms, and Therapies. Oxid Med Cell Longev, 2016, 6842568.
del Baño, M. J., Lorente, J., Castillo, J., Benavente-García, O., del Río, J. A., Ortuño, A., Quirin, K.-W., & Gerard, D. (2003). Phenolic Diterpenes, Flavones, and Rosmarinic Acid Distribution during the Development of Leaves, Flowers, Stems, and Roots of Rosmarinus officinalis. Antioxidant Activity. Journal of Agricultural and Food Chemistry, 51(15), 4247-4253.
Ding, Y., Choi, K. J., Kim, J. H., Han, X., Piao, Y., Jeong, J. H., Choe, W., Kang, I., Ha, J., Forman, H. J., Lee, J., Yoon, K. S., & Kim, S. S. (2008). Endogenous hydrogen peroxide regulates glutathione redox via nuclear factor erythroid 2-related factor 2 downstream of phosphatidylinositol 3-kinase during muscle differentiation. Am J Pathol, 172(6), 1529-1541.
El Haddad, M., Notarnicola, C., Evano, B., El Khatib, N., Blaquiere, M., Bonnieu, A., Tajbakhsh, S., Hugon, G., Vernus, B., Mercier, J., & Carnac, G. (2017). Retinoic acid maintains human skeletal muscle progenitor cells in an immature state. Cell Mol Life Sci, 74(10), 1923-1936.
El-Lakany, A. M. (2004). Chlorosmaridione; a novel chlorinated diterpene quinone methide from Rosmarinus officinalis L. Natural Product Sciences, 10(2), 59-62.
Erkan, N., Ayranci, G., & Ayranci, E. (2008). Antioxidant activities of rosemary (Rosmarinus Officinalis L.) extract, blackseed (Nigella sativa L.) essential oil, carnosic acid, rosmarinic acid and sesamol. Food Chem, 110(1), 76-82.
Faure, P., & Lafond, J. L. (1995). Measurement of plasma sulfhydryl and carbonyl groups as a possible indicator of protein oxidation. In A. E. Favier, J. Cadet, B. Kalyanaraman, M. Fontecave & J. L. Pierre (Eds.), Analysis of Free Radicals in Biological Systems, (pp. 237-248). Basel: Birkhäuser Basel.
Hanson, R. L., Lardy, H. A., & Kupchan, S. M. (1970). Inhibition of phosphofructokinase by quinone methide and alpha-methylene lactone tumor inhibitors. Science, 168(3929), 378-380.
Hirasawa, Y., Izawa, E., Matsuno, Y., Kawahara, N., Goda, Y., & Morita, H. (2007). Taxodistines A and B, abietane-type diterpenes from Taxodium distichum. Bioorg Med Chem Lett, 17(21), 5868-5871.
Hueso-Rodriguez, J. A., Jimeno, M. L., Rodriguez, B., Savona, G., & Bruno, M. (1983). Abietane diterpenoids from the root of Salvia phlomoides. Phytochemistry, 22(9), 2005-2009.
Jean, E., Laoudj-Chenivesse, D., Notarnicola, C., Rouger, K., Serratrice, N., Bonnieu, A., Gay, S., Bacou, F., Duret, C., & Carnac, G. (2011). Aldehyde dehydrogenase activity promotes survival of human muscle precursor cells. J Cell Mol Med, 15(1), 119-133.
Kitzmann, M., Bonnieu, A., Duret, C., Vernus, B., Barro, M., Laoudj-Chenivesse, D., Verdi, J. M., & Carnac, G. (2006). Inhibition of Notch signaling induces myotube hypertrophy by recruiting a subpopulation of reserve cells. J Cell Physiol, 208(3), 538-548.
Kolak, U., Kabouche, A., Ozturk, M., Kabouche, Z., Topcu, G., & Ulubelen, A. (2009). Antioxidant diterpenoids from the roots of Salvia barrelieri. Phytochem Anal, 20(4), 320-327.
Kupchan, S. M., Karim, A., & Marcks, C. (1968). Taxodione and taxodone, two novel diterpenoid quinone methide tumor inhibitors from Taxodium distichum. J Am Chem Soc, 90(21), 5923-5924.
Kuzma, L., Kaiser, M., & Wysokinska, H. (2017). The production and antiprotozoal activity of abietane diterpenes in Salvia austriaca hairy roots grown in shake flasks and bioreactor. Prep Biochem Biotechnol, 47(1), 58-66.
Kuzma, L., Wysokinska, H., Sikora, J., Olszewska, P., Mikiciuk-Olasik, E., & Szymanski, P. (2016). Taxodione and Extracts from Salvia austriaca Roots as Human Cholinesterase Inhibitors. Phytother Res, 30(2), 234-242.
Liu, Y. L., Lindert, S., Zhu, W., Wang, K., McCammon, J. A., & Oldfield, E. (2014). Taxodione and arenarone inhibit farnesyl diphosphate synthase by binding to the isopentenyl diphosphate site. Proc Natl Acad Sci USA, 111(25), E2530-2539.
Lo, A. H., Liang, Y. C., Lin-Shiau, S. Y., Ho, C. T., & Lin, J. K. (2002). Carnosol, an antioxidant in rosemary, suppresses inducible nitric oxide synthase through down-regulating nuclear factor-kappaB in mouse macrophages. Carcinogenesis, 23(6), 983-991.
Luis, J. C., & Johnson, C. B. (2005). Seasonal variations of rosmarinic and carnosic acids in rosemary extracts. Analysis of their in vitro antiradical activity. Spanish Journal of Agricultural Research, 3(1), 7.
Morel, S., Landreau, A., Nguyen, V. H., Derbre, S., Grellier, P., Pape, P. L., Pagniez, F., Litaudon, M., & Richomme, P. (2012). Preparative isolation, fast centrifugal partition chromatography purification and biological activity of cajaflavanone from Derris ferruginea stems. Phytochem Anal, 23(2), 152-158.
Moreno, S., Scheyer, T., Romano, C. S., & Vojnov, A. A. (2006). Antioxidant and antimicrobial activities of rosemary extracts linked to their polyphenol composition. Free Radic Res, 40(2), 223-231.
Newman, D. J., & Cragg, G. M. (2012). Natural products as sources of new drugs over the 30 years from 1981 to 2010. J Nat Prod, 75(3), 311-335.
Ortuno, J., Serrano, R., Jordan, M. J., & Banon, S. (2016). Relationship between antioxidant status and oxidative stability in lamb meat reinforced with dietary rosemary diterpenes. Food Chem, 190, 1056-1063.
Papuc, C., Goran, G. V., Predescu, C. N., & Nicorescu, V. (2017). Mechanisms of Oxidative Processes in Meat and Toxicity Induced by Postprandial Degradation Products: A Review. Comprehensive Reviews in Food Science and Food Safety, 16(1), 96-123.
Passerieux, E., Hayot, M., Jaussent, A., Carnac, G., Gouzi, F., Pillard, F., Picot, M. C., Bocker, K., Hugon, G., Pincemail, J., Defraigne, J. O., Verrips, T., Mercier, J., & Laoudj-Chenivesse, D. (2015). Effects of vitamin C, vitamin E, zinc gluconate, and selenomethionine supplementation on muscle function and oxidative stress biomarkers in patients with facioscapulohumeral dystrophy: A double-blind randomized controlled clinical trial. Free Radic Biol Med, 81, 158-169.
Perez-Fons, L., Garzon, M. T., & Micol, V. (2010). Relationship between the antioxidant capacity and effect of rosemary (Rosmarinus officinalis L.) polyphenols on membrane phospholipid order. J Agric Food Chem, 58(1), 161-171.
Rodriguez, B. (2003). 1H and13C NMR spectral assignments of some natural abietane diterpenoids. Magnetic Resonance in Chemistry, 41(9), 741-746.
Salucci, S., Burattini, S., Baldassarri, V., Battistelli, M., Canonico, B., Valmori, A., Papa, S., & Falcieri, E. (2013). The peculiar apoptotic behavior of skeletal muscle cells. Histol Histopathol, 28(8), 1073-1087.
Shafaei-Bajestani, N., Emami, S. A., Asili, J., & Tayarani-Najaran, Z. (2014). Anti-apoptotic effect of taxodione on serum/glucose deprivation-induced PC12 cells death. Cell Mol Neurobiol, 34(8), 1103-1109.
Shah, M. A., Bosco, S. J., & Mir, S. A. (2014). Plant extracts as natural antioxidants in meat and meat products. Meat Sci, 98(1), 21-33.
Sirocchi, V., Devlieghere, F., Peelman, N., Sagratini, G., Maggi, F., Vittori, S., & Ragaert, P. (2017). Effect of Rosmarinus officinalis L. essential oil combined with different packaging conditions to extend the shelf life of refrigerated beef meat. Food Chem, 221, 1069-1076.
Srancikova, A., Horvathova, E., & Kozics, K. (2014). Biological effects of four frequently used medicinal plants of Lamiaceae. Neoplasma, 60(6), 585-597.
Steinhubl, S. R. (2008). Why have antioxidants failed in clinical trials? Am J Cardiol, 101(10A), 14D-19D.
Sunderman, F. W., Jr., Marzouk, A., Hopfer, S. M., Zaharia, O., & Reid, M. C. (1985). Increased lipid peroxidation in tissues of nickel chloride-treated rats. Ann Clin Lab Sci, 15(3), 229-236.
Xiong, Y. L. (2017). Inhibition of hazardous compound formation in muscle foods by antioxidative phytophenols. Ann N Y Acad Sci.
Ahmed, A.S. and Ei-Emary, N.A. (1999). Biologically active abietane diterpenes from Taxodium distichum seeds. Bull Pharm Sci, Assiut Univ 22 (1), 21-25.
Búfalo, J., Cantrell, C.L., Jacob, M.R., Schrader, K.K., Tekwani, B.L., Kustova, T.S., Ali, A. and Boaro, C.S.F. (2016). Antimicrobial and antileishmanial activities of diterpenoids isolated from the roots of Salvia deserta. Planta Med 82, 131-7.
Chan, H.H., Hwang, T.L., Su, C.R., Reddy, M.V.B. and Wu, T.S. (2011). Anti-inflammatory, anticholinesterase and antioxidative constituents from the roots and the leaves of Salvia nipponica Miq. var.formosana. Phytomedicine 18, 148-50.
Eghtesadi, F., Farimani, M.M., Hazeri, N. and Valizadeh, J. (2016). Abietane and nor-abitane diterpenoids from the roots of Salvia rhytidea. SpringerPlus 5, 1068.
Esquivel, B., Flores, M., Hernandez-Ortega, S., Toscano, R.A. and Ramamoorthy, T.P. (1995). Abietane and icetexane diterpenoids from the roots of Salvia aspera. Phytochem 39(1), 139-43.
Fraga, B.M., Diaz, C.E., Guadano, A. and Gonzalez-Coloma, A. (2005). Diterpenes from Salvia broussonetii transformed roots and their insecticidal activity. J Agric Food Chem 53,5200-6.
Gandomkar, S., Yousefi, M., Habibi, Z. and As'habi, M.A. (2012). A new triterpene from Salvia xantliocheila Boiss. Nat Prod Res 26 (7), 648-53.
Habibi, Z., Cheraghi, Z., Ghasemi, S. and Yousefi, M. (2012). A new highly hydroxylated triterpene from Salvia atropatana Bunge. Nat Prod Res 26(20), 1910-3.
Ikeshiro, Y., Mase, I. and Tomita, Y. (1991). Abietane-type diterpene quinones from Salvia nipponica. Planta Med 57(6): 588.
Kasimu, R., Tezuka, Y., Tanaka, K., Gong, Z.N., Li, J.X., Basnet P., Namba, T. and Kadota, S. Liquid chromatography-mass spectrometry analysis of diterpenoid constituents of seventeen Salvia plants Journal of traditional medicines 15(2), 109-15.
Ke, R.F., Xu, S.C., Song, P., Deng, S.H., Ma, X.H. and Yang, X.Z. (2017). Crystal structure of taxodione isolated from Taxodium ascendens (B.). Acta Cryst E73, 1102-4.
Kupchan, S.M., Karim, A. and Marcks, C. (1969). Taxodione and taxodone, two novel diterpenoid quinone methide tumor inhibitors from Taxodium distichum.J Org Chem 34(12): 3912-8.
Kusumoto, N., Ashitani, T., Hayasaka, Y., Murayama, T., Ogiyama, K. and Takahashi, K. (2009). Antitermitic activities of abietane-type diterpenes from Taxodium distichum cones. J Chem Ecol 35, 635-642.
Kusumoto, N., Ashitani, T., Murayama, T., Ogiyama, K. and Takahashi K. (2010). Antifungal abietane-type diterpenes from the cones of Taxodium distichum Rich. J Chem Ecol 36,1381-6*.*
Kusumoto, N., Aburai, N., Ashitani, T., Takahashi, K. and Kimura K. (2014). Pharmacological prospects of oxygenated abietane-type diterpenoids from Taxodium distichum cones. Adv Biol Chem 4, 109-15.
Kuzma, L., Kisiel, W., Królicka, A. and Wysokinska, H. (2011). Genetic transformation of Salvia austriaca by Agrobacterium rhizogenes and diterpenoid isolation. Pharmazie 66, 904-7.
Kuzma, L., Wysokinska, H., Rozalski, M., Budzynska, A., Wieckowska-Szakielc, M., Sadowska, B., Paszkiewicz, M., Kisiel, W. and Rozalska, B. (2012). Antimicrobial and anti-biofilm properties of new taxodione derivative from hairy roots of Salvia austriaca. Phytomedicine 19, 1285-7.
Kuzma, L., Wysokinska, H., Rozalski, M., Krajewska, U. and Kisiel, W. (2012). An unusual taxodione derivative from hairy roots of Salvia austriaca. Fitoterapia 83, 770-3.
Kuzma, L. and Wysokinska, H. (2014). UPLC-DAD Determination of taxodione content in hairy roots of Salvia austriaca Jacq. Acta Chromatogr 26(4), 671-81.
Kuzma, L., Wysokinska, H., Sikora, J., Olszewska, P., Mikiciuk-Olasik, E. and Pawel Szymanski, P. (2016). Taxodione and extracts from Salvia austriaca roots as human cholinesterase inhibitors. Phytother Res 30, 234-42.
Kuzma, L., Kaiser, M. and Wysokinska, H. (2017). The production and antiprotozoal activity of abietane diterpenes in Salvia austriaca hairy roots grown in shake-flasks and bioreactor. Prep Biochem Biotechnol 47(1), 58-66.
Li, M., Zhang, J.S., Ye, Y.M. and Fang, J.N. (2000). Constituents of the Roots of Salvia prionitis. J Nat Prod 63, 139-41.
Luis, J.G. and Grille, T.A. (1993). New diterpenes from Salvia munzii: chemical and biogenetic aspects. Tetrahedron 49 (28), 6217-84.
Machumi, F., Samoylenko, V., Yenesew, A., Derese, S., Midiwo, J.O., Wiggers, F.T., Jacob, M.R., Tekwani, B.L., Khan, S.I., Walker, L.A. and Muhammad, I. (2010). Antimicrobial and antiparasitic abietane diterpenoids from the roots of Clerodendrum eriophyllum. Nat Prod Commun 5(6), 853-8.
Mirzaei, H.H., Firuzi, O., Schneider, B., Baldwin, I.T. and Jassbi, A.R. (2017). Cytotoxic diterpenoids from the roots of Salvia lachnocalyx. Rev Bras Farmacogn 27, 475-9.
Mothana, R.A., Al-Said, M.S., Al-Musayeib, N.M., El Gamal, A.A., Al-Massarani, S.M., Al-Rehaily, A.J., Abdulkader, M. and Maes, L. (2014). In vitro antiprotozoal activity of abietane diterpenoids isolated from Plectranthus barbatus Andr. Int JMol Sci 15, 8360-71.
Moujir, L., Gutierrez-Navarro, A.M., San Andres, L. and Luis, J.G. (1996).Bioactive diterpenoids isolated from Salvia mellifera. Phytother Res 10, 172-4.
Naman, C.B., Gromovsky, A.D., Vela, C.M., Fletcher, J.N., Gupta, G., Varikuti, S., Zhu, X., Zywot, E.M., Chai, H., Werbovetz, K.A., Satoskar, A.R., and Kinghorn, D. (2016). Antileishmanial and cytotoxic activity of some highly oxidized abietane diterpenoids from the bald cypress, Taxodium distichum. J Nat Prod 79, 598-606.
Sabri, N.N., Abou-Donia, A.A., Assad, A.M., Ghazy, N.M., El-Lakany, A.M., Tempesta, M.S and Sanson, D.R. (1989). Abietane diterpene quinones from the roots of Salvia verbenaca and S. lanigera. Planta Med 55(6), 582.
Salae, A.W., Rodjun, A., Karalai, C., Ponglimanont, C., Chantrapromma, S., Kanjana-Opas, A., Tewtrakul, S. and Fun, H.K. (2012). Potential anti-inflammatory diterpenes from Premna obtusifolia. Tetrahedron 68, 819-29.
Simoes, F., Michavila, A., Rodriguez, B., Garcia-Alvarez, M.C. and Hasan, M. (1986). A quinone methide diterpenoid from the root of Salvia moorcraftiana. Phytochem 25 (3), 755-6.
Tayarani-Najaran, Z., Mousavi, S.H., Tajfard, F., Asili, J., Soltani, S., Hatamipour, M. and Emami, S.A. (2013). Cytotoxic and apoptogenic properties of three isolated diterpenoids from Salvia chorassanica through bioassay-guided fractionation. Food Chem Toxicol 57, 346-51.
Tezuka, Y., Kasimu, R., Li, J.X., Basnet, P., Tanaka, K., Namba, T. and Kadota, S. (1998). Constituents of roots of Salvia deserta Schang. (Xinjiang-Danshen). Chem Pharm Bull 46 (1), 107-12.
Topçu, G. and Ulubelen, A. (1996). Abietane and rearranged abietane diterpenes from Salvia montbretii. J Nat Prod 59, 734-7.
Topçu, G., Altiner, E.N., Gozcu, S., Halfon, B., Aydogmus, Z., Pezzuto, J.M., Zhou, B.N. and Kingston, D.G.I. (2003). Studies on di- and triterpenoids from Salvia staminea with cytotoxic activity. Planta Med 69, 464-7.
Topçu, G., Kolak, U., Öztürk, M., Boga, M., Hatipoglu, S.D., Bahadori, F., Çulhaoglu, B. and Dirmenci, T. (2013). Investigation of anticholinesterase activity of a series of Salvia extracts and the constituents of Salvia staminea. The Natural Products Journal 3, 3-9. Ulubelen, A. and Tuwci, E. (1990). New diterpenes from Salvia pachystachys. J Nat Prod 53(6), 1597-9.
Ulubelen, A. and Topçu, G. (1992). New abietane diterpenoids from Salvia montbretii. J Nat Prod 55 (4), 441-4.
Ulubelen, A., Topçu, G., Chai, H.B. and Pezzuto, J.M. (1999). Cytotoxic activity of diterpenoids isolated from Salvia hypargeia. Pharm Biol 37 (2), 148-51.
Zaghloul, A.M., Gohar, A.A., Naiem, Z.A. and Abdel Bar F.M. (2008). Taxodione, a DNA-Binding compound from Taxodium distichum L. (Rich.). Z Naturforsch C 63(5-6), 355-60.
Zhang, J., Rahman, A.A., Jain, S., Jacob, M.R., Khan, S.I., Tekwani, B.L. and Ilias, M. (2012). Antimicrobial and antiparasitic abietane diterpenoids from Cupressus sempervirens. Res Rep Med Chem 2,1-6. http://dx.doi.org/10.2147/RRMC.S29902.

## Claims

1. Taxodione or a rosemary stem extract for its use to prevent and/or decrease oxidation in muscle.

2. Taxodione or a rosemary stem extract for its use according to claim 1 to prevent and/or decrease proteins oxidative degradation in muscle and/or to prevent and/or decrease accumulation of pro-oxidant molecules in muscle.

3. Taxodione or a rosemary stem extract for its use according to claim 1 or to claim 2 to prevent and/or treat loss of muscle mass and/or muscle fatigue and/or muscle wasting diseases wherein said loss of muscle mass, muscle fatigue and muscle wasting diseases are associated with oxidative stress.

4. Taxodione or a rosemary stem extract for its use according to anyone of the preceding claims wherein said rosemary stem extract comprises at least 1% w/w of taxodione.

5. Taxodione or a rosemary stem extract for its use according to anyone of the preceding claims wherein said rosemary stem extract is obtained by preparing a dry powder of rosemary stem; macerating said dry powder in an organic or hydroalcoolic solvent for at least 5 days, preferably 7 days; recovering the liquid phase and evaporating the solvent; wherein said solvent is selected amongst an alcohol, a hydrocarbon, a halogenated hydrocarbon, acetone, ethyl acetate, water or a mixture thereof.

6. Use of taxodione or a rosemary stem extract as a natural preservative agent for food product containing proteins and/or lipids.

7. Use of claim 6 wherein said food product containing proteins is selected amongst food product containing meat, non-heat treated processed meat, heat-treated processed meat, processed fish and fishery products, processed eggs and egg products, and dehydrated milk.

8. Use of claim 6 wherein said food product containing lipids is selected amongst seasoning, condiments, mustard, soups and broth sauces, fats and oils essentially free from water.

9. Use of claim 6 wherein said food product containing proteins and/or lipids is a food product containing meat selected in the group consisting of fresh meat and delicatessen.

10. Process of preparation of a food product containing meat having an improved shelf life, wherein said process comprises the step of adding taxodione or a rosemary stem extract to said food product containing meat.

11. Use of taxodione or of an extract of rosemary stem as nutritional agent for livestock for improving meat and meat-derived food product quality.

12. The use according to claim 11, for decreasing protein degradation and/or lipid peroxidation and/or improving color, flavor and/or texture stabilities of the meat or of the meat-derived food products.

13. Method for improving livestock meat quality comprising the step of adding taxodione or an extract of rosemary stem to livestock feed.
